# EUROPEAN PATENT APPLICATION

(11) **EP 0 760 247 A2**
(43) Date of publication of application: **05.03.1997**
(21) Application number: 96306040.5
(22) Date of filing: 19.08.1996
(51) Int. Cl.: B01D 53/04

(54) **Oxygen concentrator monitoring system**

(30) Priority: 30.08.1995 US 521352
(71) Applicant: DeVilbiss Health Care, Inc., Somerset, Pennsylvania 15501-0635 (US)
(72) Inventor: Frola, Frank R., Trafford, Pennsylvania 15085 (US)
(74) Representative: Waldren, Robin Michael

(57) **Abstract**

A molecular sieve oxygen concentrator is provided with sensors for monitoring the oxygen flow and concentration and the pressure drop across the molecular sieve beds. The sieve beds are switched between gas separation and gas purge cycles based on gas pressure and the cycle times are measured. Information on the settings, the maintenance history and the operation of the concentrator is stored in a digital memory. The stored information may be accessed and updated by a service person through a portable hand held unit (9). The information may be transferred to a memory in the hand held unit and later transferred to the dealer's computer or, optionally, the information may be transferred via a modem and the telephone system directly to the dealer's computer (46). More accurate information is provided for timely servicing oxygen concentrators.

## Description

### Technical Field

The invention relates to oxygen concentrators and more particularly to a system for monitoring the operation of a pressure swing molecular sieve medical oxygen concentrator.

### Background Art

Oxygen concentrators are used, for example, as a source of high purity oxygen for medical applications. An oxygen concentrator will separate nitrogen from air to produce a gas stream which consists primarily of oxygen. A pressure swing oxygen concentrator typically has at least two molecular sieve beds. Each molecular sieve bed consists of a closed cylindrical container partially filled with a sieve material, such as a zeolite, which will pass a flow of oxygen molecules while blocking or adsorbing the larger nitrogen molecules. In operation, a compressor applies filtered pressurized air through a flow control valve to an inlet port on one of the molecular sieve beds and about 95% pure oxygen flows under pressure from an outlet port on such bed. The oxygen may flow to an accumulator and then pass through a pressure regulator, an optional flow meter and a final filter to the patient.

As oxygen flows through the sieve bed, the separated nitrogen is retained in the sieve bed. After a predetermined short operating time, one or more valves are changed to apply the pressurized air to the inlet port of a second sieve bed and to vent the inlet port of the first sieve bed. A small portion of the pressurized oxygen output from the second sieve bed is delivered to the outlet port of the first sieve bed. This oxygen flows in a reverse direction through the first sieve bed to purge nitrogen and any other trapped gases from the first sieve bed. The valves are periodically reversed to alternate the sieve beds between the gas separating cycle and the trapped gas purging cycle. Preferably, an accumulator holds a volume of the concentrated oxygen under pressure to provide a continuous oxygen flow to the patient as the valves are cycled.

The molecular sieve beds may be operated on a fixed cycle time. After a particular molecular sieve bed operates to produce concentrated oxygen for a fixed time, the valves are changed to switch gas separation to a different molecular sieve bed and to purge the previously used bed. Or, preferably, a system controller monitors the pressure drop across the molecular sieve bed which is in use. As nitrogen is accumulated in the molecular sieve bed, the pressure drop will increase and the concentrated oxygen output will decrease. When a predetermined pressure drop is reached, the valve is switched. Over a long period of operation, the molecular sieve beds will lose their efficiency. If the valve is switched on a timed basis, the oxygen output from the oxygen concentrator may decrease over time. Also, the valve may be operated more often then necessary, thus shortening the operating life of the oxygen concentrator. If the valve is switched in response to the pressure drop, the valve initially will be switched less frequently. Further, valve operation will be a function of the quantity of concentrated oxygen being produced. As the molecular sieve beds lose efficiency over time, the molecular sieve beds will be cycled more frequently, but the oxygen concentrator will continue to have its rated output.

Over a period of use, an oxygen concentrator requires regular maintenance. For example, air filters periodically need to be cleaned or replaced and eventually the molecular sieve beds may need replacing. As filters and/or the molecular sieve beds become clogged, the quantity and quality of the concentrated oxygen output may deteriorate. Since medical oxygen may be life supporting, it is important that the oxygen concentrator be timely serviced. In the past, there has been no satisfactory way for the service technician to determine the actual usage time of an oxygen concentrator or when service is needed. Consequently, a conservative maintenance schedule is followed. Maintenance is scheduled more frequently then may be required in order to assure that the oxygen concentrator continues to operate at peak performance. It would be desirable for the service technician to have a way of quickly determining the number of hours that an oxygen concentrator has been operated, to determine when a condition requiring servicing has occurred and to have a way to keep uniform records for each serviced oxygen concentrator. Also, it may be desirable for the physician who has prescribed oxygen therapy to easily determine patient compliance with the prescribed therapy.

Unnecessary maintenance increases the operating cost for an oxygen concentrator. With increased emphasis by health care providers for controlling health care costs, it is desirable to provide only necessary maintenance, but to provide such maintenance in a timely manner.

### Disclosure Of Invention

The invention is directed to a method and apparatus for monitoring the operation of a medical oxygen concentrator to determine when maintenance is needed, to indicate the nature of any needed maintenance and to record various data relating to the operation of an oxygen concentrator. The oxygen concentrator includes at least two molecular sieve beds for separating oxygen from air. The sieve beds are alternately operated in an oxygen separation mode and in a nitrogen purge mode. At least one valve, preferably a two position four way valve, is used to cycle the sieve beds between the gas separation and purge cycles. The oxygen concentration and flow rate from the molecular sieve beds are monitored by a sensor and stored in a digital memory. A microprocessor controls operation of the concentrator by operating the valve in response to the sensed pressure drop across the molecular sieve bed in the oxygen separation cycle. The pressure drop is compared with a high pressure alarm level, a valve switch level and a low pressure alarm level. In the event of an excessively high pressure, an alarm signal is stored in the digital memory, an alarm may be sounded and the concentrator is switched off. Only an alarm signal is stored for a low pressure. The computer also stores in the memory the valve cycle times which indicate the gas separation time for each sieve bed. If the cycle time is outside a predetermined range, an alarm message also is stored and the concentrator is turned off. Information on the total operating time and the oxygen output pressure and/or flow rate also may be collected and stored in the memory. When the concentrator is initially set up for operation, it also may be programmed with the serial number of the concentrator, the identity of the patient, the prescribed oxygen therapy and any other desired information. The memory in the concentrator also may serve as a notebook for storing information on the concentrator setup and past maintenance.

The operational data stored in the system memory may be accessed in any of several ways. The service person may have a digital hand held unit which includes a keyboard, a memory and a display. The hand held unit is connected to a serial input/output data port on the oxygen concentrator. The hand held unit allows the service person to access and display information on the past service history of the oxygen concentrator, on usage of the oxygen concentrator and on its operation including the cause of any alarms. As service calls are made, the data from each oxygen concentrator can be stored in the hand held unit for later transfer to the dealer's computer.

The input/output port also may be connected through a modem to a telephone line for communication with a dealer's computer. The oxygen concentrator may be programmed to automatically dial the dealer's computer at specific times and to download data. When the oxygen concentrator is at the dealer's shop either prior to delivery and installation at the patient's home or for servicing, the dealer's computer may be conn3ected directly to the input/output port on the oxygen concentrator. The computer may be used to download operational data from the oxygen concentrator or to program the oxygen concentrator.

The monitoring system for an oxygen concentrator provides several advantages which reduce the operating costs for the concentrator. Improved service can be provided to the patient at a lower cost to the provider. The time for performing preventative maintenance checks is reduced, allowing the service technical to service a greater number of concentrators. Data is made available for performance tracking which will help to schedule maintenance when the unit requires service verses the prior art system of scheduling maintenance at preset time intervals. Data also is available for preparing reports for the dealer and the physician.

Accordingly, it is an object of the invention to provide a monitoring system for a molecular sieve oxygen concentrator.

Other objects and advantages of the invention will become apparent from the following detailed description of the invention and the accompanying drawings.

### Brief Description Of The Drawings

Fig. 1 is a schematic diagram of an exemplary oxygen concentrator suitable for use with the monitoring system of the invention;
Fig. 2 is a block diagram of a monitoring system for an oxygen concentrator according to the invention;
Fig. 3 is a program flow chart illustrating operation of the oxygen concentrator for switching the molecular sieve bed selection valve and for generating messages relating to the operation of the molecular sieve beds;
Figs. 4a and 4b are a program flow chart illustrating operation of the oxygen concentrator program cycle for storing and transferring data;
Fig. 5 is a plan view of a hand held portable data display and memory unit for displaying service information regarding an oxygen concentrator and for transferring data to a dealer's microcomputer; and
Figs. 6a - 6e are a program flow chart illustrating operation of the portable data display and memory unit.

### Best Mode For Carrying Out The Invention

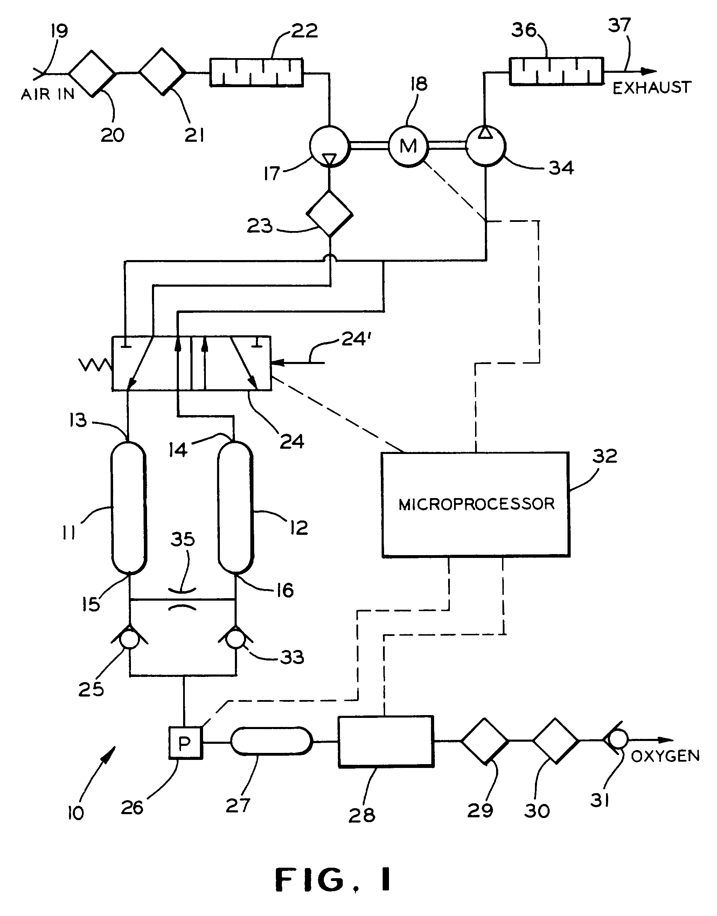

Referring to Fig. 1 of the drawings, an exemplary pressure swing molecular sieve oxygen concentrator 10 is illustrated. The oxygen concentrator 10 is of the type used to produce concentrated oxygen for medical applications, although the concentrated oxygen also may be used for other applications. The oxygen concentrator 10 includes two molecular sieve beds 11 and 12 which are partially filled with a material which will adsorb nitrogen while permitting a flow of oxygen between inlet ports 13 and 14 and outlet ports 15 and 16 on the beds 11 and 12, respectively. Various nitrogen adsorbent materials are known. Frequently, zeolite is used as the adsorbent material.

A compressor 17 driven by a motor 18 draws room air from an inlet 19 through a primary filter 20, a secondary filter 21 and a muffler 22. The air is discharged from the compressor 17 through a high efficiency filter 23 to a two position four way valve 24. The valve 24 is illustrated in its "on" or actuated position. When turned "off', the valve 24 moves to the left, as illustrated by the arrow 24'. In the illustrated position, the valve 24 applies the filtered compressed air to the inlet port 13 on the sieve bed 11. Concentrated oxygen flows from the outlet port 15, while nitrogen is retained in the bed 11. The concentrated oxygen flows through a check valve 25 and a pressure sensor 26 to an accumulator 27. The pressure sensor 26 may be adapted to measure both the concentrated oxygen pressure at the accumulator 27 and the pressure drop across the operating one of the molecular sieve beds 11 or 12. From the accumulator 27, the oxygen flows through a sensor 28 which measures the oxygen concentration, the oxygen flow rate and the oxygen temperature, through a final filter 29 and a check valve 30 to an oxygen outlet 31. The outlet 31 is connected, for example, through a suitable hose and mask or cannula (not shown) to supply concentrated oxygen to the respiratory track of a patient.

A microprocessor or computer 32 controls the operation of the oxygen concentrator 10. The microprocessor 32 is connected to control the motor 18 and the valve 24 in response to information from the pressure sensor 26 and the sensors 28. If desired, the speed of the motor 18 may be adjusted to provide a desired gas pressure and/or flow rate or a flow or pressure regulator (not shown) may be provided. As nitrogen is separated from oxygen in the sieve bed 11, the pressure drop across the sieve bed 11 increases and hence the pressure of the concentrated oxygen delivered to the accumulator 27 will decrease. When the microprocessor 32 senses a predetermined drop in the oxygen pressure or a predetermined pressure increase across the sieve bed 11 or 12, the valve 24 is operated to apply the compressed air to the inlet port 14 to the sieve bed 12. Concentrated oxygen then flows from the outlet port 16 through a check valve 33, through the pressure sensor 26 and to the accumulator 27. The accumulator 27 functions to maintain a more uniform flow pressure while the valve 24 is operated and the next sieve bed 11 or 12 comes up to pressure.

While the sieve bed 11 is operated to separate nitrogen from oxygen, the other sieve bed 12 is purged of retained nitrogen. The valve 24 connects the inlet port to the sieve bed 12 to a suction pump 34. The suction pump 34 also may be driven by the motor 18 which drives the compressor 17. The suction pump 34 reduces the pressure at the inlet port 14. A calibrated orifice 35 connects the sieve bed outlet ports 15 and 16 to permit a limited flow of concentrated oxygen from the pressurized bed 15 or 16 to the outlet port 16 or 15 of the bed being purged. The pressurized oxygen at the outlet port 16 and the reduced pressure at the inlet port 14 causes a reverse flow through the sieve bed 12 and any trapped nitrogen is purged or desorbed by the oxygen flow. The oxygen and desorbed nitrogen are discharged through a muffler 36 and an exhaust port 37 to atmosphere. Optionally, the suction pump 34 and the muffler 36 may be omitted and the valve 24 may connect the inlet port 13 or 14 of the bed 11 or 12 being purged either directly to the muffler 36 or directly to the exhaust port 37.

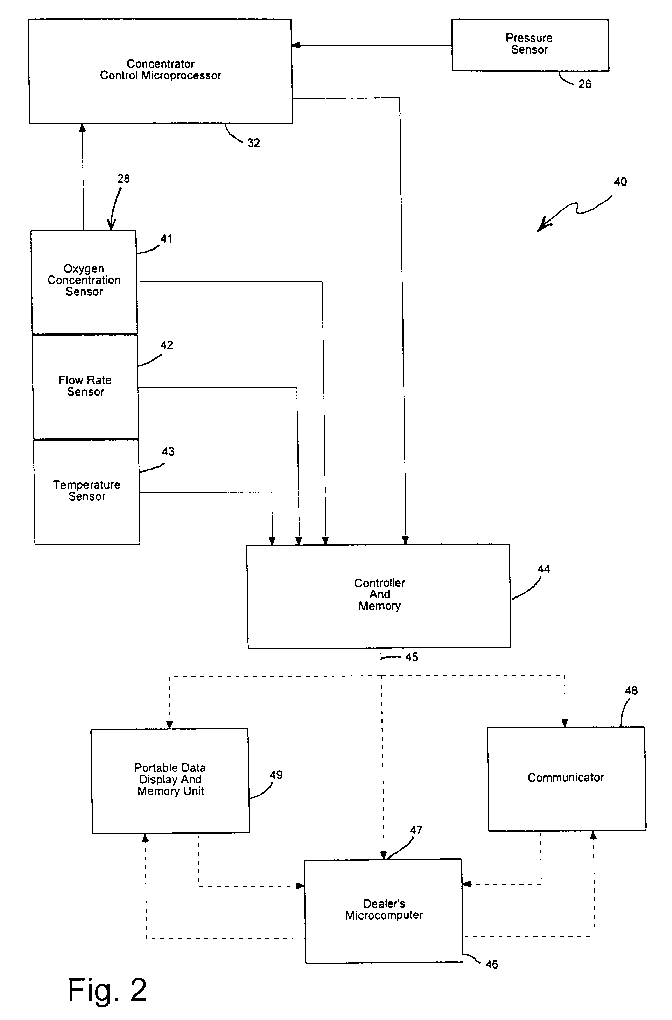

Fig. 2 is a block diagram illustrating a monitoring system 40 according to a preferred embodiment of the invention for an oxygen concentrator such as the concentrator 10 of Fig. 1. The pressure sensor 26 and the sensors 28 are shown connected to supply operational data to the concentrator control microprocessor 32. In response to this data, the microprocessor 32 operates the motor 18 and the cycling valve 24 (Fig. 1) and also measures the valve cycle time. The sensors 28 are illustrated as a single unit which includes an oxygen concentration sensor 41, an oxygen flow sensor 42 and an oxygen temperature sensor 43 which are connected to a controller and memory 44 in addition to the microprocessor 32. Preferably, the sensors 28 are combined into a single device, such as the sensor described and illustrated in United States patent 5,247,826, the disclosure of which is incorporated herein. The sensor 28 may include transducers and a microprocessor which calculates gas concentration, gas flow rate and gas temperature from information generated by the transducers. The microprocessor may be included in the controller and memory 44.

As described above, the operation of the concentrator 10 is controlled by the microprocessor 32. The microprocessor 32 controls the compressor motor 18, the valve 24 and generates messages and alarms. It continuously monitors the pressure in the accumulator tank 27 as well as the cycle times. Based upon parameters stored in non-volatile memory in the controller and memory 44, the microprocessor 32 will alarm if a system malfunction is determined by 1) the pressure being either too low or too high, or 2) the gas separation cycle time for each sieve bed 11 and 12 being either too long or too short, or 3) oxygen concentration falling below a stored set point, or 4) a microprocessor timing error or 5) a non-volatile memory malfunction. The microprocessor 32 provides a signal representing the valve states to the controller and memory 44 and digital codes representing the status of the oxygen concentrator 10.

The controller and memory 44 is the overseer of the oxygen concentrator 10. It continuously monitors the communication from the microprocessor 32 for alarm messages and checks the valve switching times. At a rate of twice per second, the controller and memory 44 measures the oxygen concentration coming out of the concentrator 10 as well as the temperature and the flow rate of the concentrated oxygen. This information is sent twice per second to a communications port 45 in a standard serial data format. At the conclusion of the data stream, the controller and memory 44 monitors the communications port 45 for commands from an external device. Oxygen concentrator operational data stored in the controller and memory 44 also may include the serial number of the oxygen concentrator 10, hours of operation, patient identification and prescription details, and service information such as a record of past services performed and a schedule of when future service will be needed.

One or more of three different communications methods and devices may be used for communicating with the oxygen concentrator 10. A dealer who sells, leases or services the oxygen concentrator 10 may have a microcomputer 46 having a port 47 which may be connected directly to the communications port 45 when initially setting up the oxygen concentrator 10 prior to delivery to the patient and when the concentrator 10 is in the shop for servicing. The dealer's microcomputer 46 may be used to keep a master record on each oxygen concentrator sold, leased or serviced and to update the patient and service data stored in the controller and memory 44.

As an alternate method for transferring data between the dealer's microcomputer 46 and the oxygen concentrator 10, the oxygen concentrator 10 may include a communicator 48 which connects the communications port 45 to a telephone line. The communicator 48 includes a microprocessor, a non-volatile memory, a real time clock for time and date stamping the stored data, a modem and a battery. The dealer's microcomputer 46 also is provided with a modem (not shown) connected to a telephone line to permit communications between the oxygen concentrator 10 and the microcomputer 46. If a communicator 48 is provided in the oxygen concentrator, it may be programmed to automatically connect to the dealer's computer 46 either at preprogrammed times to download maintenance and usage data or whenever one or more alarms are generated to send data identifying the oxygen concentrator and the nature of the alarm. The communicator 48 also may inform the dealer when routine service based on operating time is needed. The dealer then will know immediately when service is needed and will know the urgency of the needed service. The communicator 48 will reduce the service costs for the oxygen concentrator, since service will be performed only when needed and the technician will know the nature of the needed service prior to making a service call. In the past, it was necessary for the servicing technician to make regularly scheduled service calls based on an estimated usage of the oxygen concentrator. This often resulted in service calls before they were needed. If desired, each time the oxygen concentrator is turned on and off may be stored and periodically transmitted either to the dealer's computer or to the patient's physician's computer. From this information, the physician can determine patient compliance with the prescription and can confirm that the oxygen concentrator was set to administer the prescribed oxygen flow. If the patient is not responding properly to the prescribed therapy, the physician will know either that the prescription needs revising or that the patient is not complying with the prescription.

Alternately, a portable, hand held data display and memory unit 49 may be connected to the communications port 45 by a service technician during a service call. The hand held unit 49 may download data from a number of oxygen concentrators as the service technician makes service calls during a day and subsequently the data is downloaded from the hand held unit 49 into the dealer's microcomputer 46 at the end of the day to update the dealer's records. When data is downloaded into the dealer's computer, the data may be stored in file formats that are compatible with popular spread sheet programs, word processor programs and/or data base programs. These programs can be used for generating reports, trend analysis, or patient/unit records. Further, the hand held unit 49 may be used by the technician to display data such as alarm messages, the service record and other information relating to the oxygen concentrator 10. For example, the service technician may be informed that a particular filter needs to be replaced. Once the filter is replaced, the service technician may update the data stored in the controller and memory 44, using a keyboard on the hand held unit 49, to show that the filter has been replaced. The keyboard also may be used to modify the information stored in the oxygen concentrator, such as the service record, or the patient prescription.

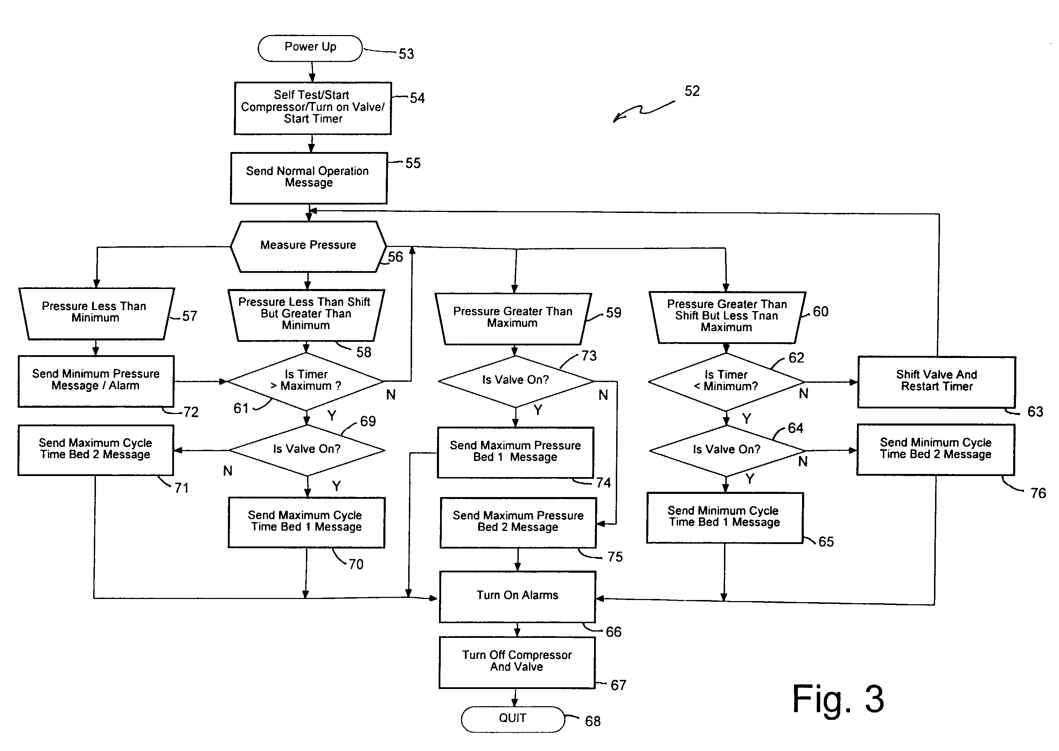

Referring now to Fig. 3, a flow chart 52 illustrates operation of the microprocessor 32 in the oxygen concentrator 10 of Fig. 1 for switching the molecular sieve bed selection valve 24 and for generating messages relating to the operation of the molecular sieve beds. Initially, the flow chart 52 is entered at a power up terminal 53. A selftest cycle is automatically run, the compressor motor 18 is started, the valve 24 is turned on and a valve cycle timer is started at a block 54. The valve cycle timer is a clock in the microprocessor 32 which measures the elapse time between changing the position of the valve 24. Hence, the cycle timer measures the gas separation operating cycle time for each of the molecular sieve beds 11 and 12. A "normal operation" message then is generated at a block 55. The pressure drop across the operating molecular sieve bed 11 or 12, as measured by the sensor 26, is then read at a block 56. Pressure data is supplied from the block 56 to four blocks 57-60. The controller and memory 44 (Fig. 2) stores several pressures which are used to control operation of the oxygen concentrator 10. A minimum acceptable oxygen pressure is stored, a shift pressure threshold for the valve 24 is stored and a maximum acceptable oxygen pressure is stored. The block 57 establishes an output if the measured oxygen pressure is less than the stored minimum pressure. The block 58 establishes an output if the measured oxygen pressure is less than the shift pressure but greater than the minimum pressure. The block 59 establishes an output if the measured oxygen pressure is greater than the stored maximum pressure. The block 60 establishes an output if the measured oxygen pressure is greater than the stored shift pressure but less than the stored maximum pressure.

During normal operation of the oxygen concentrator 10 with the valve 24 on, the measured pressure will be less than the shift pressure but greater than the minimum pressure until it is time to regenerate the sieve bed 11. The block 58 will apply an output to a decision block 61 which asks if the valve cycle timer has exceeded a stored maximum cycle time. If not, the microprocessor returns to the block 32 and again measures the pressure at the block 56. Once the pressure exceeds the shift pressure while remaining below the stored maximum pressure, the block 60 applies an output to a decision block 62 which asks if the timer is less than the minimum cycle time. If the cycle timer is not less than the minimum, a block 63 shifts the valve 24, restarts the cycle timer and returns to the block 56 for again measuring the pressure. If the cycle timer was less than the minimum cycle time at the block 62, a decision block 64 asks if the valve 24 is on or off. If the valve 24 is on, a "minimum cycle time bed 1" message is generated at a block 65 and if the valve 24 is off, a "minimum cycle time bed 2 " message is generated at a block 76. In either case, an alarm is turned on at a block 66, the compressor motor 18 and the valve 24 are turned off at a block 67 and the program quits at a terminal 68.

As indicated above, if the concentrated oxygen pressure is determined to be less than the shift pressure but greater than the minimum pressure, the block 61 asks if the valve cycle timer has exceeded its maximum time. If the maximum cycle time has been exceeded, a block 69 asks if the valve 24 is on. If the valve 24 is on, the block 70 sends a "maximum cycle time bed 1" message, an alarm is turned on at the block 66, the compressor and valve are turned off at the block 67 and the program quits at the terminal 68. If the valve 24 was determined to be off at the decision block 69, a block 71 sends a "maximum cycle time bed 2" message, an alarm is turned on at the block 66, the compressor motor 18 and the valve 24 are turned off at the block 67 and the program quits at the terminal 68.

If the measured oxygen pressure was determined at the block 57 to be less than the stored minimum pressure, a block 72 sends a "minimum pressure" message and an alarm. However, the oxygen concentrator 10 is not shut off in response to only a minimum pressure message, since the measured pressure will be below the minimum whenever the oxygen concentrator 10 is initially turned on and may briefly drop below the minimum immediately after the valve 24 is shifted. From the block 72, the program advances to the decision block 61 which compares the cycle timer with the stored maximum cycle time. From the block 62, the program will either return to the pressure measurement block 56 or will proceed to establish a "maximum cycle time" message at one of the blocks 70 or 71.

If the measured pressure is determined to exceed the stored maximum pressure at the block 59, a decision block 73 asks if the valve 24 is on. If so, a block 74 sends a "maximum pressure bed 1" message. If the valve 24 is off, a block 75 sends a "maximum pressure bed 2" message. After a message is sent at either block 74 or block 75, an alarm is turned on at the block 66, the compressor motor 18 and the valve 24 are turned off at the block 67 and the program quits at the terminal 68.

The various messages generated by the program illustrated in the flow chart 52 are stored in the controller and memory 44 of Fig. 2. If a communicator 48 is provided, the communicator 48 may be programmed to dial the telephone number for the dealer's microcomputer 46 and to transmit both data identifying the patient and oxygen concentrator which requires service and the message established by the microprocessor 32. If a portable data display and memory unit 49 is used by a service technician, the messages may be displayed and/or the messages and other data relating to the oxygen concentrator 10 may be downloaded into the memory for transfer at a later time to the dealer's microcomputer 46.

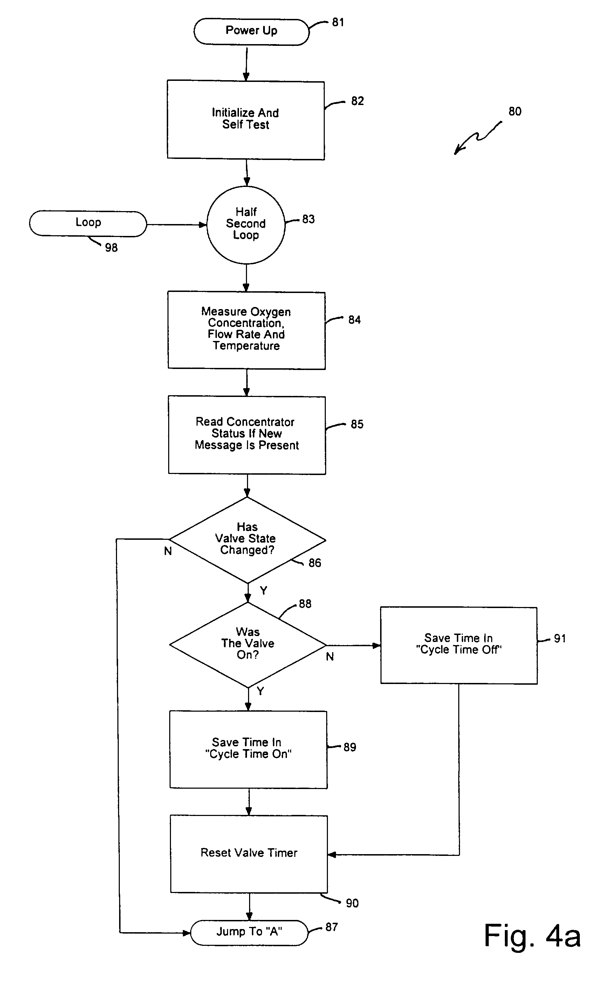

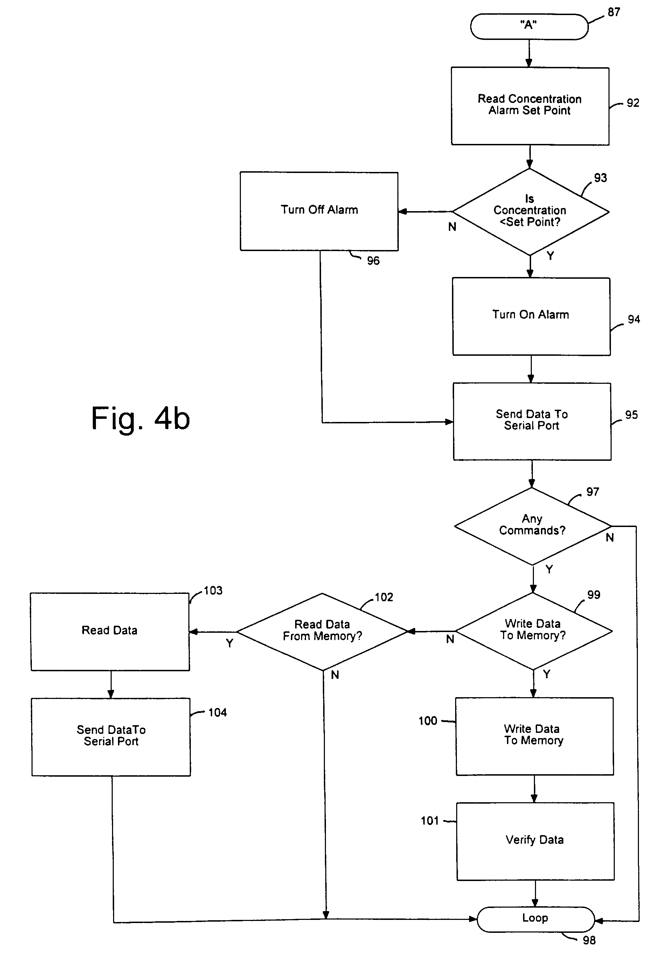

Figs. 4a and 4b are a program flow chart 80 which illustrate the process for storing and transferring data. On power up, the cycle is began at a terminal 81. At a block 82, the microprocessor in the controller and memory 44 is initialized and self tests are run. The process then goes to a half second loop timer 83 which includes a timer for automatically recycling through the flow chart 80 every one half second. From the loop timer 83, the oxygen concentration percentage, the concentrated oxygen flow rate and the concentrated oxygen temperature are measured at a block 84. The operating status of the concentrator then is read at a block 85 if any new message is present. A decision block 86 asks if the valve status has changed since the last program loop cycle. If not, the program jumps through a terminal 87 to "A" in Fig. 4b. If the valve status has changed, a decision block 88 asks if the valve 24 was "on". If the valve was "on", the "on" time is saved as "Cycle Time On" at a block 89, the valve timer is reset at a block 90 and the program jumps via the terminal 87 to "A" in Fig. 4b. If the block 88 determines that the valve was "off', a block 91 saves the "off" time as "Cycle Time Off", the valve timer is reset at the block 90 and the program jumps via the terminal 87 to "A" in Fig. 4b.

From the terminal 87 in Fig. 4b, the alarm set point for the oxygen concentration is read at block 92. A decision block 93 then asks if the concentration is less than the set point. If so, a low concentration alarm is turned of at a block 94 and the data is sent to the serial port (45 in Fig. 2) at a block 95. If the oxygen concentration was not less than the set point at the block 93, the low oxygen concentration alarm is turned off at a block 96 and the data is sent to the serial port at the block 95. After the data is sent to the serial port, a decision block 97 asks if there are any commands. If not, the program returns through a loop terminal 98 to the loop timer 83 in Fig. 4a. If there were commands at the decision block 97, a decision block 99 asks if the command was to write data to the non volatile memory. If so, the data is written at a block 100, verified at a block 101 and the program returns through the loop terminal 98 to the loop timer 83 in Fig. 4a. If decision block 99 determines that the command was not to write data, a decision block 102 asks if data was to be read from the non volatile memory. If not, the program returns to the cycle timer 83 through the loop terminal 98. If it was determined at the block 102 that data was to be read, the data is read at a block 103 and sent to the serial port at a block 104. The program then returns through the loop 98 to the cycle timer 83. Data will be read whenever requested by a device connected to the communications port 45, such as by the portable data display and memory unit 49.

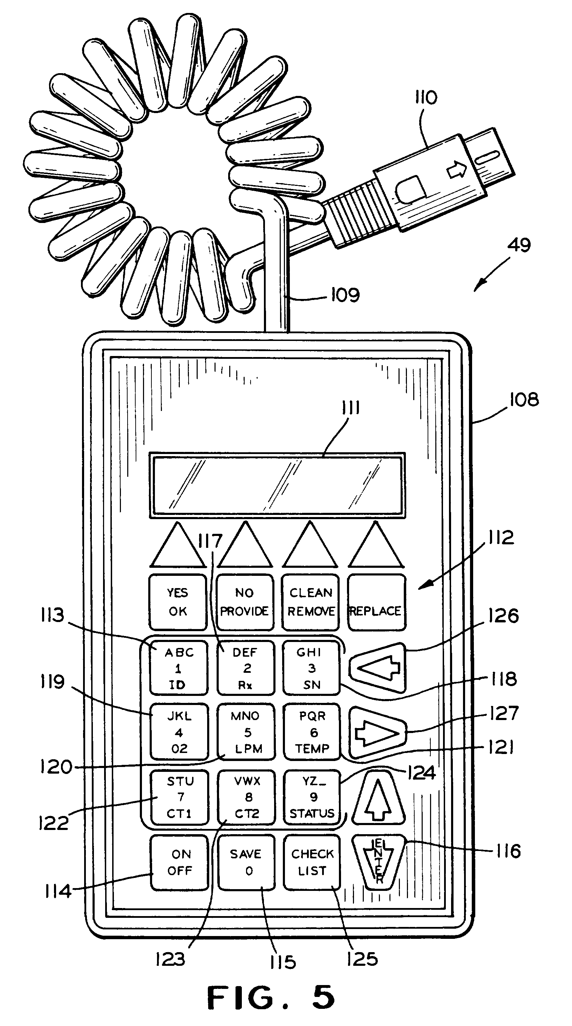

Fig. 5 is a plan view showing a portable data display and memory unit 49. Preferably, the unit 49 is of a sufficiently small size to be easily held in the hand of the service technician during use. The unit 49 includes an internal non volatile memory (not shown) to which data may be written and read. The memory may be, for example, an EEPROM. The unit 49 has a housing 108 from which a serial data cord 109 extends. The cord 109 terminates at a connector 110 which is adapted to connect to the serial communications port 45 on the oxygen concentrator monitoring system 40 of Fig. 2. A digital data display 111 is located on the housing 108 along with a keyboard 112 for entering data and for requesting the display of data. Many of the keys on the keyboard 112 may have multiple functions. For example, a key 113 is labeled "ABC 1 ID" When the unit 49 is first turned on with a key 114, the operator is asked to enter his or her initials to record the service technician's identification. If the first initial of the technician is an "A", the key 113 is pushed once. If the first initial is a "B", the key 113 is pushed twice, or if it is a "C" it is pushed three times. After the first initial is displayed, left and right arrow keys 126 and 127 may be pushed to move the cursor for entering and/or changing the remaining initials. An "Enter" key 116 then is pushed to save the initial and the remaining two initials are similarly entered.

Once the initials are entered, the display 111 indicates "Task Completed". If a "Save" key 115 is pushed, the operator is asked to enter the accumulated hours of operation of the oxygen concentrator as indicated on an hour meter. If the "Enter" key 116 is then pushed, all data relating to operation of the oxygen concentrator, including the entered operating hours, and the date and time are stored in a non volatile memory in the controller and memory 44. The key 113 may be used to display the patient identification and, if necessary, the patient identification may then be changed. A key 117 may be used to select "Rx" for displaying and modifying the patient prescription. Both a rest flow rate and, if desires, an activity flow rate may be entered. A key 118 may be used to display and reprogram the serial number of the oxygen concentrator 10. The serial number will be changed only when the controller and memory 44 has been replaced. A key 119 is pushed to display the oxygen concentration, a key 120 is pushed to display the oxygen flow rate in liters per minute and a key 121 is pushed to display the oxygen temperature. A key 122 displays the cycle time for the molecular sieve bed 11 I and a key 123 displays the cycle time for the molecular sieve bed 12. A key 124 displays the status of the oxygen concentrator 10 and causes any alarm messages to be displayed. A key 125 displays a record for the oxygen concentrator including a maintenance check list. The technician may move down through the check list and enter a record of any maintenance performed and times when maintenance will be required. The oxygen concentrator record will be stored in the internal memory in the unit 49. An exemplary record for an oxygen concentrator is shown in Table I. Preferably, the memory in the portable unit 49 is sufficiently large to store records for all oxygen concentrators serviced by a technician during at least one day and may cover a longer period such as one week. Periodically, the information stored in the unit 49 is downloaded into the dealer's computer 46. This information is used for maintaining a service record on each oxygen concentrator and for scheduling future maintenance. By having an accurate record on each oxygen concentrator, the dealer can efficiently provide service only when needed, thus reducing the cost for supplying oxygen to the patient.

**TABLE I**

| Concentrator Data: | |
|---|---|
| Serial No. | M12345DS |
| Hours of Operation: | 1534 |
| Concentration | 94.35 (%) |
| Flow Rate: | 4.1 (LPM) |
| Temperature: | 78.3 (°F) |
| Cycle Time 1: | 10.9 (sec) |
| Cycle Time 2: | 10.8 (sec) |
| Error Message: | Unit Running OK |
| Audible Alarm | OK |
| Visual Alarm | OK |
| Check LPM | 3.9 |
| O2 Analyze | 94.2 (%) |
| Check LPM | 5.0 |
| O2 Minimum | 93.8 (%) |
| Exterior Filter | Replaced |
| Long Life Intake Filter | Yes |
| Intake Filter | OK |
| Check Battery | OK |
| Cabinet Clean | Cleaned |
| Cannula/Mask | Provided 2 |
| O2 Tubing | Replaced 1 |
| Humidifier | Removed 1 |
| O2 Port Fitting | OK |
| O2 Tank | Provided |
| Total Tank Qty | 3 |
| O2 Tank Size | D |
| O2 Conserver | Yes |
| O2 Cart Bag | No |
| No Smoke Sign | Provided 2 |
| Provider Info | Provided 1 |
| Instruction Guide | OK |
| Preposition Unit | No |
| Unit Repaired | No |
| Unit Replaced | No |

| Patient Usage: | |
|---|---|
| Rx LPM Resting | 4.0 |
| Rx Hours/Day | 10 |
| Rx Humidification | No |
| Usage Check | 358 hours/34 days = 10.5 hours/day |

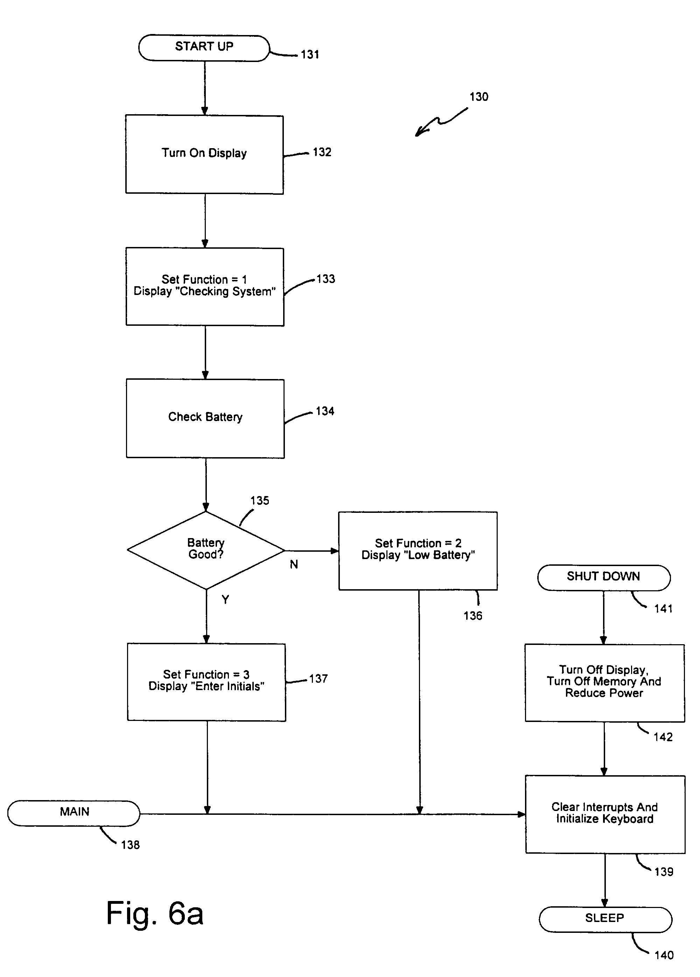

Figs. 6a to 6e form a flow chart 130 illustrating the preferred operation of the portable data display and memory unit 49. Referring first to Fig. 6a, the unit 49 is first connected either to the serial port 45 of the oxygen concentrator 10 or to a serial port on the dealer's microcomputer 46. The flow chart 130 is initiated at a START UP terminal 131, as will be discussed in greater detail below. The display 111 (Fig. 5) is turned on at a block 132. The unit has a number of preset functions. Exemplary functions are listed below in Table II. After the display is initially turned on, the function is set to 1 at a block 133 and the display 111 is set to indicate "Checking System" while the operation of the unit 49 is automatically checked. The battery is then checked at a block 134 and a "good battery? query is asked at a decision block 135. If the battery if not good, the function is set to 2 and the display 111 is set to indicate "Low Battery" at a block 136. If the battery is determined to be good, the function is set to 3 and the display 111 is set to indicate "Enter Initials" at a block 137. In either case, the program proceeds to a MAIN terminal 138 which connects to a block 139 where interrupts are cleared and the keyboard 112 is initialized. The program then goes to a SLEEP terminal 140 and waits for the next command either from the keyboard or through the serial cable 109 from an oxygen concentrator or a microcomputer. The portion of the program in Fig. 6a also has an input terminal 141 called SHUT DOWN. Whenever a SHUT DOWN command is received, the display and memory are turned off and the power is reduced at a block 142. The program then proceeds through the block 139 to the SLEEP terminal 140. If the program goes to the SLEEP terminal 140 via the MAIN terminal 138, the display 11 and the memory in the unit 49 will remain on, while if the SLEEP terminal 140 is reached via the SHUT DOWN terminal 141, they are turned off.

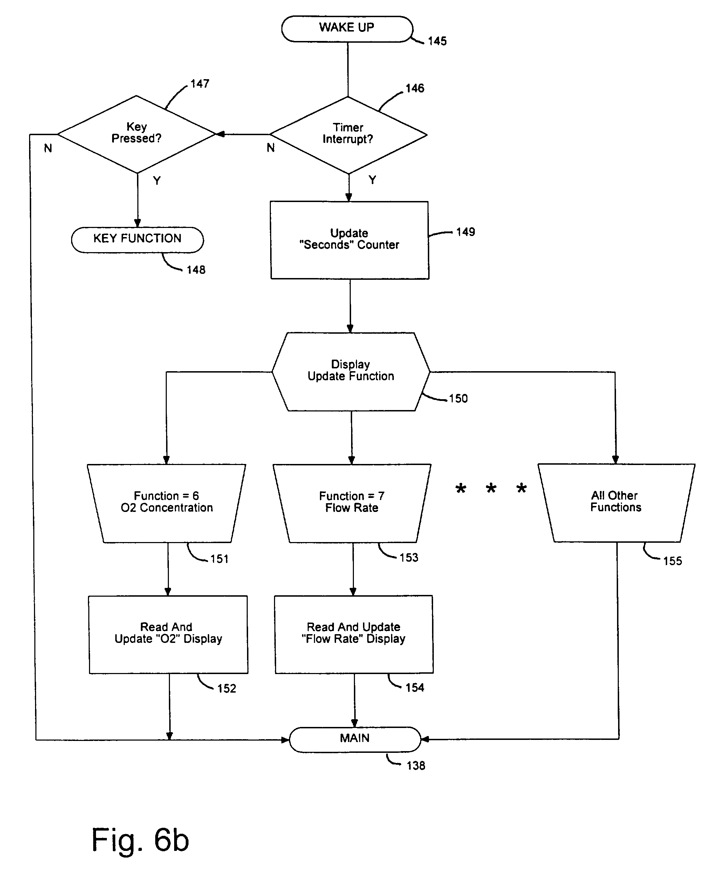

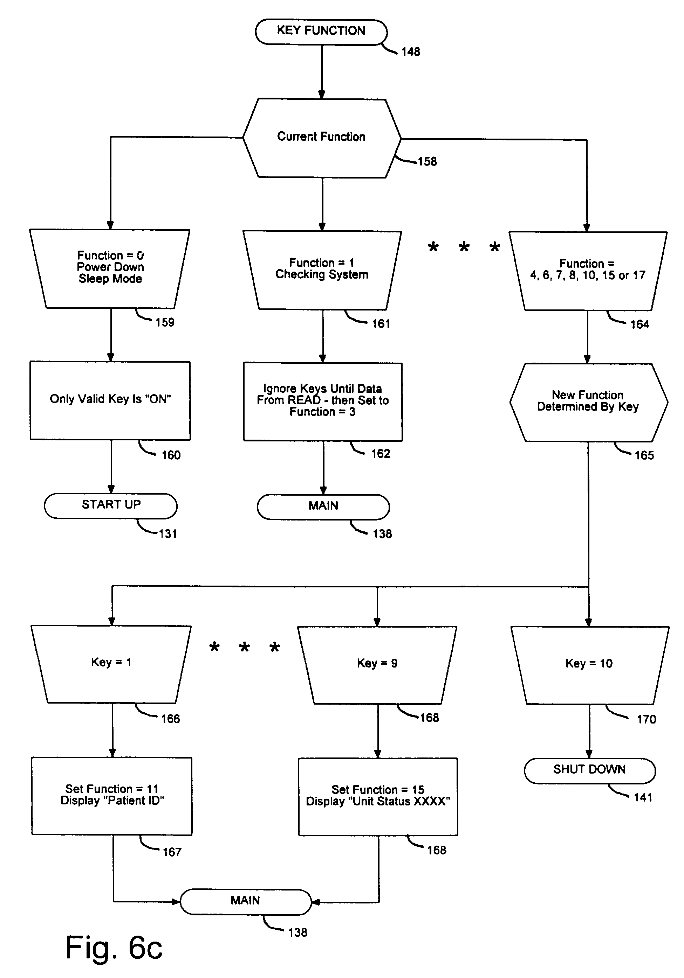

The unit 49 enters the SLEEP mode to conserve power. It will remain in the SLEEP mode until it receives an interrupt. If the memory and display unit 49 remains on, the pressing of any key on the keyboard 112 will generate an interrupt which will cause the program to enter a WAKE terminal 145 in Fig. 6b. If the SLEEP mode was entered via the SHUT DOWN terminal 141, the power ON key 114 must be pushed. A timer (not shown) also will periodically wake the unit 49 from the power on SLEEP mode. At a decision block 146, it is determined if the unit 49 woke because of a timer interrupt. If not, a decision block 147 asks if a key was pressed. If so, the program jumps to a KEY FUNCTION terminal 148 in Fig. 6c. If a key was not pressed, the program jumps to the main terminal 138 in Fig. 6a.

**TABLE II**

| Function | Description |
|---|---|
| 0 | Power Down Mode |
| 1 | Checking System |
| 2 | Low Battery Display |
| 3 | Input Operators Initials |
| 4 | Task Completed/Next Function Mode |
| 5 | Display Unit Serial Number |
| 6 | Display Oxygen Concentration |
| 7 | Display Flow Rate |
| 8 | Display Temperature In °F. |
| 9 | Display Cycle Time For Bed 11 |
| 10 | Display Cycle Time For Bed 12 |
| 11 | Display Patient ID |
| 12 | Display Prescription Flow Rate |
| 13 | Display Prescription Hours/Day |
| 14 | Display Prescription Humidification |
| 15 | Display Concentrator Status Message |
| 16 | Input Hours Of Operation |
| 17 | Store Data |

If there was a timer interrupt at the decision block 146, the "seconds" counter is updated at a block 149 and the unit prepares to update the display function at a block 150. If a display function is present and it is, for example, Function 6, the program proceeds through a block 151 to a block 152 where the current oxygen concentration is read from a connected oxygen concentrator and is displayed. The program then jumps to the MAIN terminal 138 in Fig. 6a. If, for example, the display function 7 is present, the program proceeds from the block 150 through a block 153 to a block 154 where the flow rate data is read from a connected oxygen concentrator and is displayed. The program then jumps to the MAIN terminal 138 in Fig. 6a. Similar blocks (not shown) are provided for the other display functions. If the function is not a display function, the program passes through a block 155 and jumps to the MAIN terminal 138 in Fig. 6a.

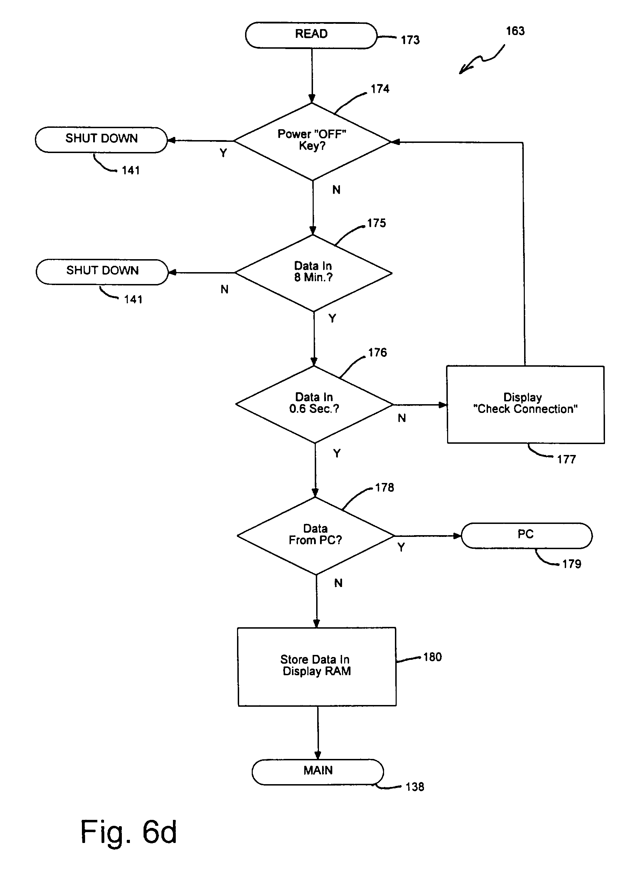

If a key was pressed at the decision block 147, the program jumps to the KEY FUNCTION terminal 148 in Fig. 6c. The program then looks at the current function at a block 158. If the function is set to 0 for the power down sleep mode, the program passes through a block 159. The program will respond at a block 160 only to the "ON" key 114 and jump to the START UP terminal 131 in Fig. 6a. If the function was set to 1 for checking the system, the program proceeds from the block 158 through a block 161 to a block 162. The keys are ignored until data is received from a READ loop 163 in Fig. 6d. When data is received, the function is set to 3 and the program then jumps to the MAIN terminal 138 in Fig. 6a. Similar blocks (not shown) are provided for each of the remaining functions other than those which are marked on the keypad 112 (Fig. 5).

It will be noted in Fig. 5 that in addition to other markings, the key 113 is marked with the function "ID" (patient ID), the key 118 is marked with the function "Rx" (prescription), the key 118 is marked with the function "SN" (concentrator serial number), the key 119 is marked with the function "02" (oxygen concentration), the key 120 is marked with the function "LPM" (liters per minute oxygen flow rate), the key 121 is marked with "Temp" (oxygen temperature °F), the key 122 is marked with the function "CT1" (cycle time for sieve bed 11), the key 123 is marked with the function "CT2" (cycle time for the sieve bed 12), the key 124 is marked with the function "Status" (alarm messages), and the key 115 is marked with "SAVE". Referring again to Fig. 6c and to Table II, if the function was set to any of Functions 4, 6, 8, 10, 15 or 17, the program passes through blocks 164 and 165. If, for example, the "1" key or key 113 is pressed, the program passes through a block 166 to a block 167 where the function is set to 11 and the display is set to show "Patient ID. The program then jumps to the MAIN terminal 138. Or, if the "9" key or key 124 is pushed, the program passes through a block 168 and the function is set to 15 and the display is set to show "Unit Status XXXXX" at a block 169. The program then jumps to the MAIN terminal 138. Similar blocks (not shown) are provided for the other function keys on the keyboard 112. If the power ON/OFF key 114 was pushed, the program passes through a block 170 and jumps to the SHUT DOWN terminal 141 in Fig. 6a.

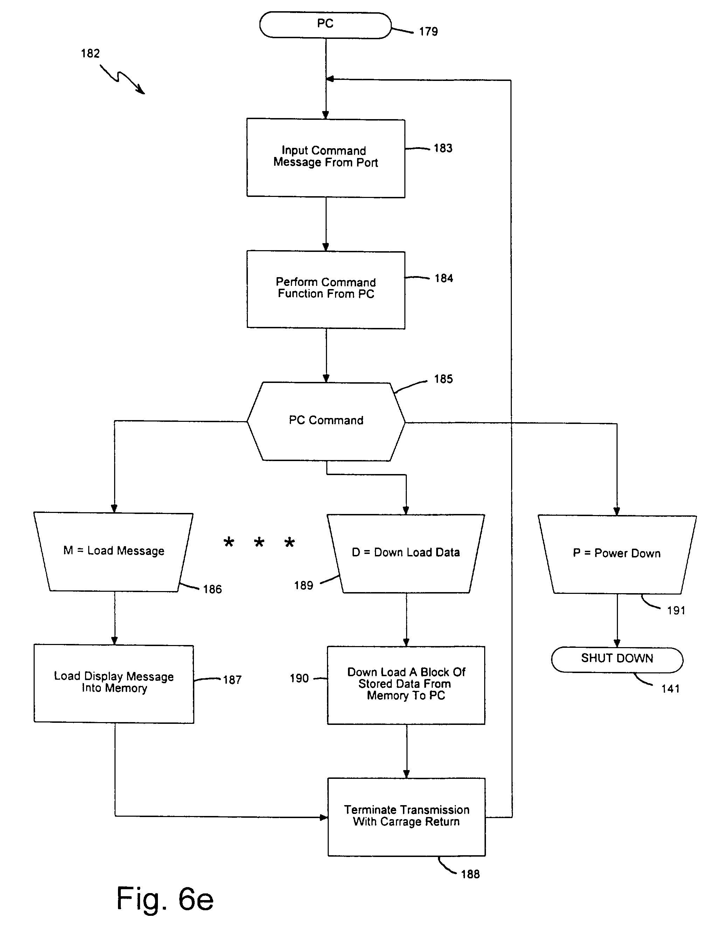

If data was called for from the READ loop 163, the program enters a READ terminal 173 in Fig. 6d. A decision block 174 then asks if the power off key 114 was pushed. If so, the program jumps to the SHUT DOWN terminal 141. If not, a decision block 175 asks if there has been any data received in the last 8 minutes. If not, the program again jumps to the SHUT DOWN terminal 141 to extend the battery life. If data has been received in the previous 8 minutes, a decision block 176 asks if it was received in the last 0.6 seconds. If not, a block 177 causes the display to indicate "Check Connection" and the program returns to the block 174. It should be noted that the oxygen concentrator program updates data and sends the data to the serial port 45 every 0.5 seconds. The dealer's microprocessor will have a similar or a faster program cycle time. If data was received within the previous 0.6 seconds, the program then asks at a decision block 178 if the data was from the dealer's microcomputer. If so, the program jumps to Fig. 6e via a PC terminal 179. If not, the data from the oxygen concentrator is stored is a random access memory at a block 180 and the program jumps to the MAIN terminal 138.

**TABLE III**

| Command | Description |
|---|---|
| M | Load Message Into Memory From PC |
| N | Read Message From Memory To PC |
| R | Read Byte From Memory To PC |
| W | Write Byte To Memory From PC |
| S | Set Time In Second Counter |
| T | Read Time To PC |
| P | Power Down |
| D | Down Load Data From Memory To PC |
| ? | Send Error Message To PC |

Fig. 6e shows a program loop 182 for communicating with the dealer's microcomputer 46. From the PC terminal 179, input command messages are read at a block 183 and any functions based on commands from the dealer's microcomputer 46 are performed at a block 184. Table III shows exemplary commands from the microcomputer 46. From the block 184, the program passes through a PC Command block 185 If, for example, the microcomputer 46 gives an "N" command to load a message, the program passes through a block 186 to a block 187 where the display message is loaded from the dealer's microcomputer 46 into an EEPROM memory in the unit 49. The transmission then is terminated with a carriage return at a block 188 and the program returns to the block 183. This loop is repeated for additional commands other than a power down command. Thus, if the next command is a "D" or down load command, the program passes from the block 185 through a block 189 to a block 190 where a block of data stored in the EEPROM memory in the unit 49 is down loaded to the microprocessor 46. Additional blocks (not shown) for the other commands are provided, as needed. If the command at the block 185 is a "P" or power down command, the program passes through a block 191 and jumps to the SHUT DOWN terminal 141 in Fig. 6a.

Having the capability to modify the operation of the oxygen concentrator provides several benefits not previously available. The capability of programming operating limits for the oxygen concentrator allows the manufacturer to use the same circuitry for different capacity oxygen concentrators. In the past, it was necessary to use different circuitry for controlling different capacity concentrators. Also, operation of the oxygen concentrator is easily modified for different markets. For example, the oxygen concentrator may be designed for operation from different voltage sources available in different countries. Changing the voltage level and frequency can affect the speed of the compressor motor which will affect the operating pressures. This in turn can change the cycle times for the molecular sieve beds. It is a simple matter to reprogram the alarm limits for the molecular sieve bed cycle times based upon the available power source. In the past, any alarm limits had to be set very broad to prevent false alarms caused by environmental changes. The cycle times could be affected, for example, by altitude and other factors. The alarm limits now can be set more closely to the actual normal operation of the oxygen concentrator for each installation.

It also should be appreciated that multiple alarm limits may be easily provided. A first alarm message may be generated when a first limit is exceeded to indicate that non urgent service should be scheduled. In this event, the oxygen concentrator is not shut down. A second alarm limit may generate an urgent message and shut down operation of the oxygen concentrator when broader limits are exceeded. The program operation for such multiple alarm limits can be easily understood by referring to Fig. 3. When the pressure was less than a minimum limit, an alarm message was established at block 72 and the oxygen concentrator continued to operate. A narrow limit range for the sieve bed cycle times and/or the maximum pressure may be set in a similar way to establish alarm messages without shutting down the concentrator.

It will be appreciated that various modifications and changes may be made to the above described preferred embodiment of a monitoring system for pressure swing oxygen concentrators without departing from the spirit and the scope of the following claims.

## Claims

1. A method for monitoring the operation of an oxygen concentrator comprising the steps of:
a) storing in a memory in said oxygen concentrator data on the operation of said oxygen concentrator;
b) attaching a portable memory and display unit to said oxygen concentrator;
c) transferring oxygen concentrator operation data from said oxygen concentrator memory to a memory in said portable memory and display unit;
d) selectively displaying operation data from said oxygen concentrator memory on said attached portable memory and display unit; and
e) selectively transferring oxygen concentrator operation data from said portable memory and display unit memory to a computer located remote from said oxygen concentrator.

2. A method for monitoring the operation of an oxygen concentrator, as set forth in claim 1, and wherein said oxygen concentrator is monitored by measuring the gas separation cycle time for each of two molecular sieve beds which are alternately switched between a gas separation cycle and a gas purge cycle in response to measured pressure and comparing said monitored cycle times for each molecular sieve bed with stored minimum and maximum cycle times.

3. A method for monitoring the operation of an oxygen concentrator, as set forth in claim 2, and further including the step of establishing a cycle time alarm message when the measured time of a gas separation cycle is outside a predetermined time range.

4. A method for monitoring the operation of an oxygen concentrator, as set forth in claim 3, and further including the steps of storing in a memory a predetermined minimum gas separation cycle time, and wherein said cycle time alarm message is established in response to a comparison of the measured gas separation cycle time and the stored minimum gas separation cycle time.

5. A method for monitoring the operation of an oxygen concentrator, as set forth in claim 3, and further including the steps of storing in a memory a minimum gas pressure, a sieve bed cycle gas pressure and a maximum gas pressure, wherein switching of said sieve bed from a gas separation cycle to a gas purge cycle is in response to a comparison of the measured gas pressure and the stored sieve bed cycle gas pressure, and further including the steps of establishing a maximum pressure alarm message when the measured gas pressure exceeds the stored maximum gas pressure and establishing a minimum pressure alarm message when the measured gas pressure falls below the stored minimum gas pressure.

6. A method for operating an oxygen concentrator, as set forth in claim 5, and further including the step of stopping operation of said oxygen concentrator in response to at least one of a cycle time alarm message and a maximum pressure alarm message.

7. A method for operating an oxygen concentrator, as set forth in claim 5, and further including the steps of transferring any alarm message from the oxygen concentrator to a computer located remote from said oxygen concentrator.

8. A method for operating an oxygen concentrator, as set forth in claim 7, and wherein said alarm message is transferred to said computer by storing said computer by writing said alarm message to said memory in said portable memory and display unit, and writing said stored alarm message from said portable memory and display unit into a memory in said computer.

9. A method for operating an oxygen concentrator of the type having a gas separation sieve bed which is alternately operated in gas separation cycle and a gas purge cycle, said method comprising the steps of:
a) switching operation of said sieve bed from a gas separation cycle to a gas purge cycle in response to a measured gas pressure;
b) measuring the time of the gas separation cycle for said sieve bed; and
c) establishing a cycle time alarm message when the time of said gas separation cycle is outside a predetermined cycle time range.

10. A method for operating an oxygen concentrator, as set forth in claim 9, and further including the steps of storing in a memory a predetermined minimum gas separation cycle time, and wherein said cycle time alarm message is established in response to a comparison of the measured gas separation cycle time and the stored minimum gas separation cycle time.

11. A method for operating an oxygen concentrator, as set forth in claim 10, and further including the steps of storing in a memory a predetermined maximum gas separation cycle time, and wherein said cycle time alarm message is established in response to a comparison of the measured gas separation cycle time and the stored minimum and maximum gas separation cycle times.

12. A method for operating an oxygen concentrator, as set forth in claim 11, and further including the steps of storing in a memory a minimum gas pressure, a sieve bed cycle gas pressure and a maximum gas pressure, wherein switching of said sieve bed from a gas separation cycle to a gas purge cycle is in response to a comparison of the measured gas pressure and the stored sieve bed cycle gas pressure, and further including the steps of establishing a maximum pressure alarm message when the measured gas pressure exceeds the stored maximum gas pressure and establishing a minimum pressure alarm message when the measured gas pressure falls below the stored minimum gas pressure.

13. A method for operating an oxygen concentrator, as set forth in claim 12, and further including the step of stopping operation of said oxygen concentrator in response to at least one of a cycle time alarm message and a maximum pressure alarm message.

14. A method for operating an oxygen concentrator, as set forth in claim 12, and further including the steps of transferring any alarm message from the oxygen concentrator to a computer located remote from said oxygen concentrator.

15. A method for operating an oxygen concentrator, as set forth in claim 14, and wherein said alarm message is transferred to said computer by programming said oxygen concentrator to connect to said remote computer in response to an alarm and to transfer information identifying said oxygen concentrator and the alarm to said remote computer.

16. A method for operating an oxygen concentrator, as set forth in claim 13, and wherein said alarm message is transferred to said computer by storing said computer by writing said alarm message to a portable memory, and writing said stored memory from said portable memory into a memory in said computer.

17. A monitoring system for an oxygen concentrator, comprising means in said oxygen concentrator for generating and storing data indicative of the operation of said oxygen concentrator, a portable data storage and display unit adapted to connect to said oxygen concentrator, said storage and display unit including means for selectively indicating operation data from said oxygen concentrator and memory means for storing operation data from said oxygen concentrator, a microcomputer located remote from said oxygen concentrator and having a port adapted to connect to said portable data storage and display unit, and means for downloading oxygen concentrator data from said portable data storage and display unit into said microcomputer.

18. A monitoring system for an oxygen concentrator, as set forth in claim 17, and wherein said memory means in said portable memory and display unit is adapted to store operation data from a plurality of oxygen concentrators, and wherein said means for downloading oxygen concentrator data downloads operation data for a plurality of oxygen concentrators from said portable data storage and display unit to said microcomputer.
